(19) Europäisches Patentamt / European Patent Office / Office européen des brevets

(11) **EP 3 631 416 B1**

(12) **FASCICULE DE BREVET EUROPEEN**

(45) Date de publication et mention de la délivrance du brevet:
**29.12.2021 Bulletin 2021/52**

(21) Numéro de dépôt: **18723872.0**

(22) Date de dépôt: **18.05.2018**

(51) Int Cl.:
*G01N 15/14* (2006.01)     *G01N 21/45* (2006.01)
*G03H 1/08* (2006.01)     *G03H 1/04* (2006.01)
*G03H 1/00* (2006.01)     *G01N 15/10* (2006.01)

(86) Numéro de dépôt international:
**PCT/EP2018/063085**

(87) Numéro de publication internationale:
**WO 2018/215337 (29.11.2018 Gazette 2018/48)**

(54) **PROCÉDÉ D'ANALYSE DE MICROORGANISMES**

VERFAHREN ZUR ANALYSE VON MIKROORGANISMEN

METHOD FOR ANALYSING MICROORGANISMS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.05.2017 FR 1754535**

(43) Date de publication de la demande:
**08.04.2020 Bulletin 2020/15**

(73) Titulaire: **Commissariat à l'Energie Atomique et aux Energies Alternatives**
**75015 Paris (FR)**

(72) Inventeurs:
- **BORDY, Thomas**
  **38000 Grenoble (FR)**
- **CIONI, Olivier**
  **38100 Grenoble (FR)**
- **DEFORCEVILLE, Camille**
  **46120 Anglars (FR)**
- **MANDULA, Ondrej**
  **38000 Grenoble (FR)**

(74) Mandataire: **Le Goaller, Christophe**
**Innovation Competence Group**
**310 avenue Berthelot**
**69372 Lyon Cedex 08 (FR)**

(56) Documents cités:
**WO-A1-2016/097092     WO-A1-2016/151248**
**WO-A1-2016/151249**

- **ALBORZ FEIZI ET AL: "Rapid, portable and cost-effective yeast cell viability and concentration analysis using lensfree on-chip microscopy and machine learning", LAB ON A CHIP, vol. 16, no. 22, 1 janvier 2016 (2016-01-01), pages 4350-4358, XP055446711, ISSN: 1473-0197, DOI: 10.1039/C6LC00976J cité dans la demande**

**Description**

**DOMAINE TECHNIQUE**

Le domaine technique de l'invention est la caractérisation de microorganismes, notamment la caractérisation de levures ou de bactéries.

**ART ANTERIEUR**

L'utilisation de microorganismes tels des levures ou des bactéries, ou leurs dérivés, trouve de nombreuses applications dans de nombreux domaines. Dans le domaine de l'agroalimentaire, par exemple, l'utilisation de levures est répandue dans différents secteurs, tels la boulangerie, la production de vin, la brasserie ou encore la fabrication de produits laitiers. L'application de levures ou de bactéries concerne de nombreux aliments par le biais de probiotiques, ces derniers étant par exemple ajoutés à des céréales ou à des aliments pour animaux. En dehors de l'agroalimentaire, de nombreux domaines industriels peuvent mettre en œuvre des microorganismes. Il s'agit par exemple de l'agriculture ou de l'horticulture, avec le développement de produits phytosanitaires ou de fertilisants plus respectueux de l'environnement, ou encore de la production de biocombustibles obtenus à partir de végétaux. D'autres applications concernent le domaine de la pharmacie et du diagnostic médical.

A des fins de contrôle qualité, l'étape de caractérisation de tels microorganismes constitue un maillon essentiel de la chaîne de production. Des contrôles microbiologiques sont fréquemment utilisés, sur des échantillons prélevés dans des milieux de culture, de façon à détecter et dénombrer les microorganismes vivants. La mise en culture sur des boîtes de Pétri est toujours très utilisée, mais comporte certains inconvénients, en particulier la préparation, la durée de l'analyse et l'impossibilité de détecter des microorganismes vivants et non cultivables.

Des techniques par dénombrement direct ont été développées, en utilisant par exemple des marqueurs de viabilité aptes à faire varier les propriétés optiques de microorganismes de façon différente selon qu'ils sont morts ou vivants. De tels marqueurs peuvent agir sur la couleur ou une propriété de fluorescence des microorganismes examinés. Mais l'étape de détection, effectuée au microscope, est généralement longue, le champ d'observation étant faible.

La publication Feizi "Rapid, portable and cost effective yeast cell viability and concentration analysis using lensfree on-chip microscopy and machine learning", Lab Chip, 2016, 16, 4350-4358, décrit une méthode de caractérisation de levures *Saccharomyces cerevisiae,* basé sur la microscopie sans lentille. Le dispositif présenté est simple, et permet de discriminer les levures mortes des levures vivantes en offrant un champ d'observation élevé. Du bleu de méthylène est préalablement mélangé au milieu de culture dans lequel baignent les levures examinées. Le procédé comporte l'acquisition d'une image du milieu de culture par un capteur d'image, et l'application d'un opérateur de propagation holographique à l'image acquise en considérant de multiples distances de propagation, de façon à effectuer une focalisation numérique, ce qui permet d'établir une distance dite optimale. Lorsque la distance optimale est obtenue, chaque levure est classifiée selon une catégorie vivante ou morte en fonction d'un indicateur établi à partir d'une image reconstruite à ladite distance optimale.

Dans d'autres documents, on a cherché à caractériser des cellules tout en évitant le recours à des marqueurs exogènes. Par exemple, le document WO2016/151249 décrit un procédé de d'analyse de cellules, disposées dans un milieu de culture, sans marquage. L'adjonction d'un marqueur est considérée, dans ce document comme pouvant avoir des conséquences sur le développement des cellules. Le document WO2016/151248 décrit un procédé pour identifier des particules, par exemple des particules sanguines, en évitant également un marquage préalable de ces dernières. Le document WO2016/097092 décrit un procédé d'imagerie selon une configuration défocalisée, pour identifier un microorganisme, à partir d'images défocalisées par rapport à un plan de mise au point d'un système optique.

Les inventeurs ont proposé une alternative à la méthode décrite dans la publication Feizi, précédemment citée, de façon à effectuer une classification entre des microorganismes morts et vivants selon un procédé simple et peu coûteux en temps de calcul. Par ailleurs, comme décrit dans la description, le procédé permet une analyse plus aboutie des microorganismes vivants.

**EXPOSE DE L'INVENTION**

Un premier objet de l'invention est un procédé d'analyse de microorganismes, les microorganismes étant disposés dans un échantillon, l'échantillon comportant un marqueur de viabilité, apte à modifier une propriété optique des microorganismes de façon différente selon qu'ils sont morts ou vivants, le procédé comportant les étapes suivantes :

a) illumination de l'échantillon à l'aide d'une source de lumière, la source de lumière émettant une onde lumineuse incidente se propageant vers l'échantillon selon un axe de propagation;
b) acquisition, à l'aide d'un capteur d'image, d'une image de l'échantillon, formée dans un plan de détection, l'échantillon étant disposé entre la source de lumière et le capteur d'image, chaque image étant représentative d'une onde lumineuse dite d'exposition, à laquelle est exposé le capteur d'image sous l'effet de l'illumination ;

le procédé étant caractérisé en ce qu'il comprend également les étapes suivantes :

c) détermination de positions radiales de différents microorganismes dans un plan parallèle au plan de détection, chaque position radiale étant associée à un microorganisme ;
d) à partir de l'image acquise lors de l'étape b), application d'un opérateur de propagation, de façon à calculer au moins une grandeur caractéristique de l'onde lumineuse d'exposition, à chaque position radiale déterminée lors de l'étape c), et à une pluralité de distances du plan de détection;
e) formation d'un profil, représentant une évolution de la grandeur caractéristique calculée lors de l'étape d) selon un axe parallèle à l'axe de propagation et passant par chaque position radiale déterminée lors de l'étape c), chaque profil étant associé à un microorganisme ;
f) en fonction de chaque profil formé lors de l'étape e), classification entre les microorganismes morts et vivants.

Par grandeur caractéristique, on entend par exemple un module ou une phase de l'onde lumineuse d'exposition, ou leur combinaison.

Par application d'un opérateur de propagation à partir d'une image, on entend que l'opérateur de propagation est appliqué à ladite image ou à une image résultant d'une transformation de ladite image, par exemple une racine carrée de ladite image, ainsi qu'une éventuelle normalisation de l'image.

L'étape f) peut comporter une identification des microorganismes morts.

Selon un mode de réalisation, le procédé comporte suite à l'étape f), une étape g) d'analyse de la faculté du microorganisme à se diviser, l'étape g) comportant les sous-étapes suivantes pour au moins un microorganisme considéré comme vivant lors de l'étape f):

gi) obtention d'une première image d'observation de l'échantillon, à un premier instant, l'image d'observation comportant des régions d'intérêt respectivement associées à des microorganismes, et détection d'une région d'intérêt associée audit microorganisme ;
gii) acquisition d'une image de l'échantillon à un deuxième instant, postérieur au premier instant, et obtention d'une deuxième image d'observation de l'échantillon à partir de l'image acquise au deuxième instant ;
giii) détection, sur la deuxième image d'observation, d'une région d'intérêt correspondant au microorganisme ;
giv) comparaison des régions d'intérêt détectées lors des sous-étapes gi) et giii) ;
gv) détermination de la faculté du microorganisme à se diviser en fonction de la comparaison effectuée lors de la sous-étape giv).

L'étape g) permet alors d'identifier, parmi les microorganismes identifiés comme étant vivants, les microorganismes viables et non cultivables. Elle peut être appliquée à chaque microorganisme considéré comme vivant suite à l'étape f).

Lors des sous-étapes gi) et gii), la première image d'observation et la deuxième image d'observation peuvent être obtenues par application d'un opérateur de propagation respectivement à partir d'une image acquise au premier instant et à partir d'une image acquise au deuxième instant. La première image d'observation peut résulter de l'image acquise lors de l'étape b).

L'intervalle temporel entre le premier instant et le deuxième instant peut être compris entre 5 heures et 70 heures.

Selon un mode de réalisation, lors de l'étape d) l'opérateur de propagation est appliqué à partir de l'image acquise, lors de l'étape b), selon une pluralité de distances de propagation, de façon à obtenir une pile d'images complexes.

Selon un autre mode de réalisation, l'étape d) comporte les sous-étapes suivantes :

di) application d'un opérateur de propagation, à partir de l'image acquise lors de l'étape b) de façon à calculer une image complexe, dite image de référence, représentative de l'échantillon, dans un plan de référence ;
dii) application d'un opérateur de propagation à l'image de référence, de façon à obtenir des images complexes, dites images complexes secondaires, à différentes distances du plan de référence selon l'axe de propagation, les images complexes secondaires et l'image de référence formant une pile d'images complexes ;
diii) détermination d'une position radiale de microorganismes à partir des images de la pile d'images complexes obtenue lors de la sous-étape dii).

Selon ce mode de réalisation, la première image d'observation peut être issue de l'image de référence, en étant par exemple l'image du module ou l'image de la phase de l'image de référence.

L'étape c) peut être mise en œuvre à partir d'une image complexe de la pile d'images complexes résultant de l'étape d), ou à partir de l'image complexe de référence, en considérant le module ou la phase de ladite image complexe.

Selon un mode de réalisation, le marqueur de viabilité induit une coloration des microorganismes lorsqu'ils sont morts, selon une bande spectrale de coloration. Lors de l'étape a), l'illumination de l'échantillon est effectuée selon une bande spectrale d'illumination, la bande spectrale d'illumination ne comportant pas tout ou partie de la bande spectrale de coloration.

Le procédé peut comporter une des caractéristiques suivantes, prises isolément ou selon les combinaisons techniquement réalisables :

- dans l'étape d), la grandeur caractéristique est déterminée à partir du module ou de la phase de l'onde lumineuse d'exposition, à chaque distance du plan de détection.
- L'étape f) comporte une classification de chaque profil selon une première classe, correspondant à des profils caractéristiques de microorganismes vivants et une deuxième classe, correspondant à des profils caractéristiques de microorganismes morts. La classification de chaque profil peut être effectuée en fonction de sa forme ou d'une valeur maximale ou d'une valeur minimale du profil.
- Aucune optique de formation d'image n'est disposée entre l'échantillon et le capteur d'image.
- Un système optique de formation d'image est disposé entre l'échantillon et le capteur d'image, le système optique présentant un plan focal objet, l'échantillon s'étendant dans un plan de l'échantillon, le plan de l'échantillon étant décalé par rapport au plan focal objet.

Un autre objet de l'invention est un dispositif pour l'analyse de microorganismes disposés dans un échantillon, le dispositif comportant :

- une source de lumière apte à émettre une onde lumineuse incidente se propageant vers l'échantillon;
- un capteur d'image;
- un support, configuré pour maintenir l'échantillon entre la source de lumière et le capteur d'image;

un processeur, configuré pour recevoir une image de l'échantillon acquise par le capteur d'image et à mettre en œuvre les étapes c) à f) d'un procédé selon le premier objet de l'invention.

Selon un mode de réalisation, aucune optique de grossissement ou de formation d'image ne s'étend entre le capteur d'image et l'échantillon, lorsque ce dernier est maintenu sur le support. Selon un autre mode de réalisation, un système optique de formation d'image est disposé entre l'échantillon et le capteur d'image, le système optique présentant un plan focal objet, l'échantillon s'étendant dans un plan de l'échantillon, le dispositif étant agencé de telle sorte que le plan de l'échantillon est décalé par rapport au plan focal objet.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

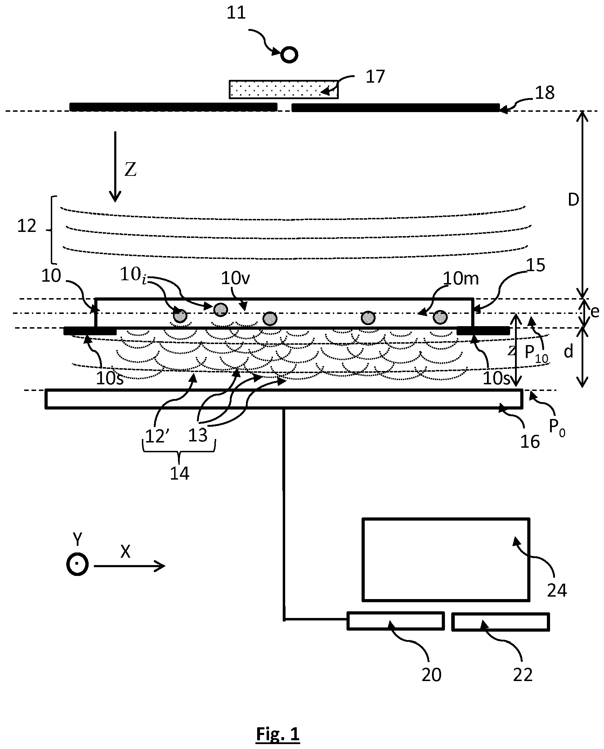

La figure 1 représente un exemple de dispositif selon l'invention.

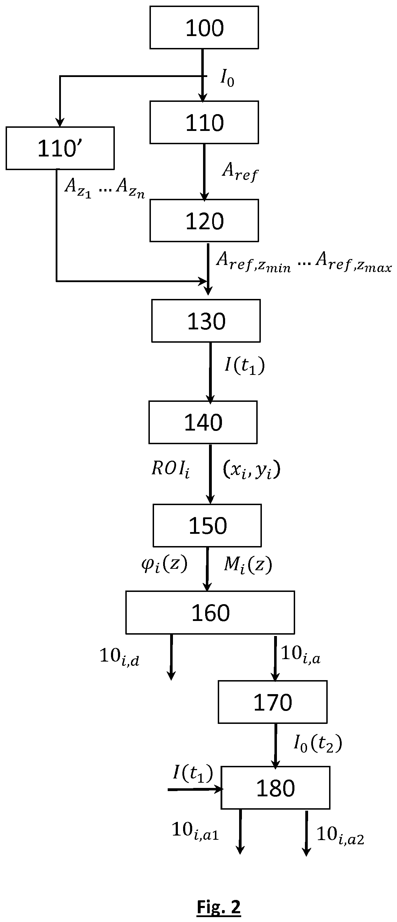

La figure 2 illustre les principales étapes d'un procédé permettant d'identifier des microorganismes vivants ainsi que, parmi ces derniers, des microorganismes viables non cultivables.

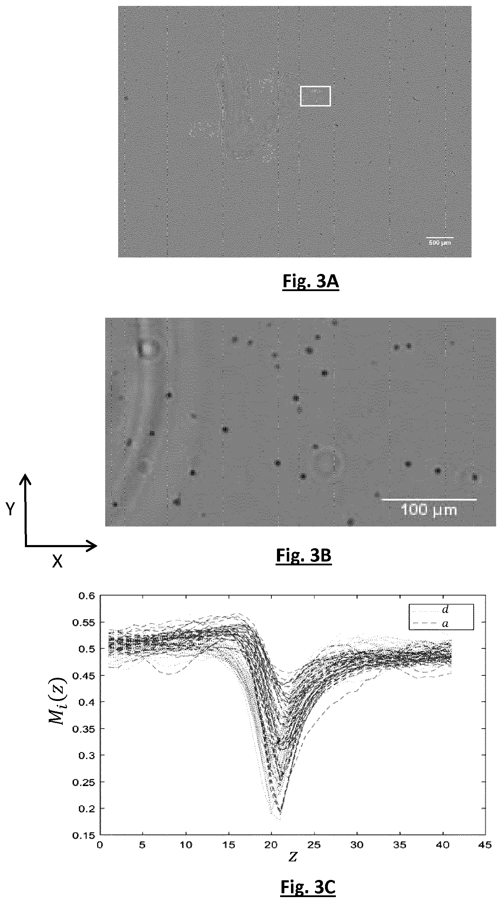

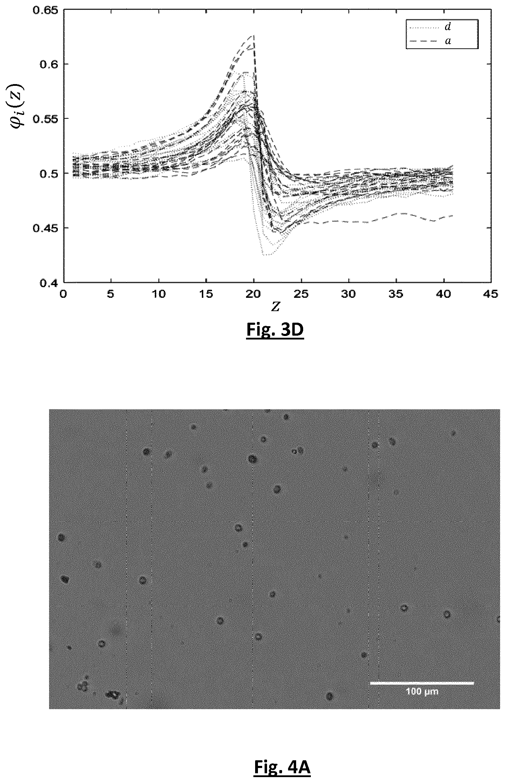

La figure 3A est une image du module d'une image complexe reconstruite à partir d'une image acquise, à un premier instant, d'un échantillon. Cette image a été obtenue au cours d'un essai expérimental réalisé en utilisant un échantillon comportant des levures. La figure 3B est détail de la figure 3A. Les figures 3C et 3D représentent respectivement des profils d'amplitude et de phase d'une onde lumineuse reconstruite à partir de l'image de la figure 3B, chaque profil passant par une position radiale, dans le plan du capteur d'image, à laquelle est associée une levure.

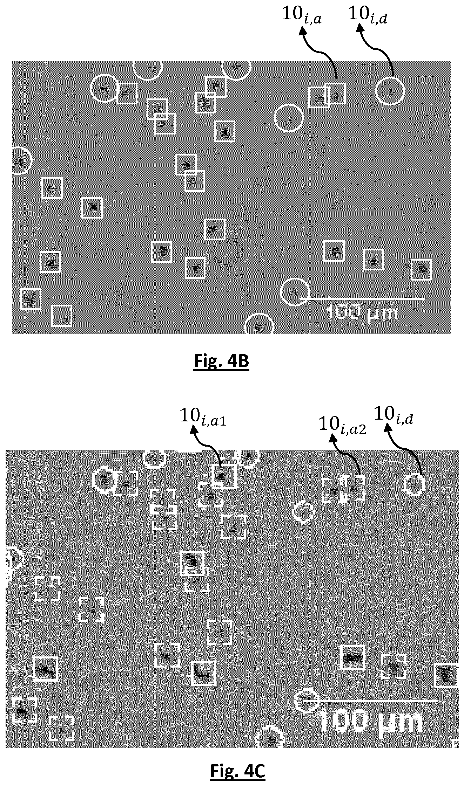

La figure 4A représente une observation au microscope de levures. La figure 4B montre, sur une partie de l'image représentée sur la figure 3B, les levures considérées comme mortes et celles considérées comme vivantes, en mettant en œuvre l'invention. Cette image correspond à l'état des levures à un premier instant. La figure 4C montre une image obtenue par reconstruction holographique à partir d'une image acquise, à un deuxième instant. Elle représente l'état des levures au deuxième instant. La comparaison entre les figures 4B et 4C permet l'identification, parmi les levures vivantes, de levures viables et non cultivables.

**EXPOSE DE MODES DE REALISATION PARTICULIERS**

La figure 1 représente un exemple de dispositif selon l'invention. Une source de lumière 11 est apte à émettre une onde lumineuse 12, dite onde lumineuse incidente, se propageant en direction d'un échantillon 10, selon un axe de propagation Z. L'onde lumineuse est émise selon une bande spectrale d'illumination $\Delta\lambda$.

L'échantillon 10 est un échantillon que l'on souhaite caractériser. Il comprend notamment un milieu 10m dans lequel baignent des micro-organismes $10_i$. Le milieu 10m est généralement un milieu de culture, comportant des nutriments permettant le développement des microorganismes. Par microorganisme, on entend notamment une levure, une bactérie, une spore, un champignon ou une cellule, qu'il s'agisse d'une cellule eucaryote ou procaryote, ou une microalgue.

L'échantillon comporte également un marqueur de viabilité $10_v$, ce dernier étant apte à modifier une propriété optique d'un microorganisme $10_i$ de façon différente selon que le microorganisme est vivant ou mort. Comme évoqué en lien avec l'art antérieur, par modification de l'aspect visuel, on entend par exemple une modification de la couleur du microorganisme. L'utilisation de tels marqueurs de viabilité est bien connue. Il peut s'agir du bleu de méthylène, ou du bleu trypan. La modification de la propriété optique peut également être une modification de l'intensité d'une lumière de fluorescence émise par un microorganisme analysé en utilisant par exemple un indicateur de viabilité, parfois désigné par le terme marqueur fluorogène, des exemples de tels marqueurs étant décrits dans WO9855861A1, ou encore dans la publication Kwolek-Mirek M "comparison of methods used for assessing the viability and vitality of yeast cells", FEMS Yeast Res 14 (2014) 1068-1079.

Dans l'exemple décrit ci-après, l'indicateur de viabilité $10_v$ est du bleu de méthylène. Sous son effet, les microorganismes morts se colorent selon une bande spectrale de coloration $\Delta\lambda'$, en l'occurrence le bleu, tandis que les microorganismes vivants restent translucides.

L'échantillon 10 est, dans cet exemple, contenu dans une chambre fluidique 15. La chambre fluidique 15 est par exemple une chambre fluidique de type Gene Frame® d'épaisseur e=250 $\mu$m. L'épaisseur e de l'échantillon 10, selon l'axe de propagation, varie typiquement entre 10 $\mu$m et 1 cm, et est de préférence comprise entre 20 $\mu$m et 500 $\mu$m. L'échantillon s'étend selon un plan $P_{10}$, dit plan de l'échantillon, perpendiculaire à l'axe de propagation Z. Il est maintenu sur un support 10s à une distance d d'un capteur d'image 16. La concentration de microorganismes peut varier entre 500 par microlitre et 5000 par microlitre.

La distance D entre la source de lumière 11 et la chambre fluidique 15 est de préférence supérieure à 1 cm. Elle est de préférence comprise entre 2 et 30 cm. Avantageusement, la source de lumière, vue par l'échantillon, est considérée comme ponctuelle. Cela signifie que son diamètre (ou sa diagonale) est préférentiellement inférieur au dixième, mieux au centième de la distance entre la chambre fluidique 15 et la source de lumière. Sur la figure 1, la source de lumière est une diode électroluminescente. Elle est généralement associée à diaphragme 18, ou filtre spatial. L'ouverture du diaphragme est typiquement comprise entre 5 $\mu$m et 1 mm, de préférence entre 50 $\mu$m et 500 $\mu$m. Dans cet exemple, le diaphragme est fourni par Thorlabs sous la référence P150S et son diamètre est de 150 $\mu$m. Le diaphragme peut être remplacé par une fibre optique, dont une première extrémité est placée face à la source de lumière 11 et dont une deuxième extrémité est placée en regard de l'échantillon 10. Le dispositif représenté sur la figure 1 comporte également un diffuseur 17, disposé entre la source de lumière 11 et le diaphragme 18. L'usage d'un tel diffuseur permet de s'affranchir de contraintes de centrage de la source de lumière 11 par rapport à l'ouverture du diaphragme 18. La fonction d'un tel diffuseur est de répartir le faisceau lumineux, produit par une source de lumière élémentaire 11 selon un cône d'angle a. De préférence, l'angle de diffusion a varie entre 10° et 80°. Alternativement, la source de lumière peut être une source laser, telle une diode laser. Dans ce cas, il n'est pas utile de lui associer un filtre spatial ou un diffuseur.

De préférence, la bande spectrale d'émission $\Delta\lambda$ de l'onde lumineuse incidente 12 a une largeur inférieure à 100 nm. Par largeur de bande spectrale, on entend une largeur à mi-hauteur de ladite bande spectrale.

Selon un mode de réalisation, la source de lumière 11 comporte plusieurs sources de lumière élémentaires $11_k$, chacune étant apte à émettre une onde lumineuse incidente $12_k$ dans une bande spectrale $\Delta\lambda_k$. De préférence, les bandes spectrales $\Delta\lambda_k$ des différentes sources de lumière $11_k$ sont différentes les unes des autres.

L'échantillon 10 est disposé entre la source de lumière 11 et le capteur d'image 16 précédemment évoqué. Ce dernier s'étend de préférence parallèlement, ou sensiblement parallèlement au plan $P_{10}$ selon lequel s'étend l'échantillon. Le terme sensiblement parallèlement signifie que les deux éléments peuvent ne pas être rigoureusement parallèles, une tolérance angulaire de quelques degrés, inférieure à 20° ou 10° étant admise. Dans cet exemple, l'échantillon s'étend selon un plan XY, perpendiculaire à l'axe de propagation Z.

Le capteur d'image 16 est apte à former une image $I_0$ de l'échantillon 10 selon un plan de détection $P_0$. Dans l'exemple représenté, il s'agit d'un capteur d'image comportant une matrice de pixels, de type CCD ou un CMOS. Le plan de détection $P_0$ s'étend de préférence perpendiculairement à l'axe de propagation Z de l'onde lumineuse incidente 12. La distance d entre l'échantillon 10 et la matrice de pixels du capteur d'image 16 est préférentiellement comprise entre 50 $\mu$m et 2 cm, de préférence comprise entre 100 $\mu$m et 2 mm.

On remarque, dans ce mode de réalisation, l'absence d'optique de grossissement ou de formation d'image entre le capteur d'image 16 et l'échantillon 10. Cela n'empêche pas la présence éventuelle de microlentilles de focalisation au niveau de chaque pixel du capteur d'image 16, ces dernières n'ayant pas de fonction de grandissement de l'image acquise par le capteur d'image, leur fonction étant d'optimiser l'efficacité de détection.

Sous l'effet de l'onde lumineuse incidente 12, les microorganismes $10_i$ présents dans l'échantillon peuvent engendrer une onde diffractée 13, susceptible de produire, au niveau du plan de détection $P_0$, des interférences, en particulier avec une partie de l'onde lumineuse incidente 12' transmise par l'échantillon. Par ailleurs, l'échantillon peut absorber une partie de l'onde lumineuse incidente 12. Ainsi, l'onde lumineuse 14, transmise par l'échantillon, et à laquelle est exposé le capteur d'image 16, désignée par le terme "onde d'exposition", peut comprendre :

- une composante 13 résultant de la diffraction de l'onde lumineuse incidente 12 par chaque microorganisme de l'échantillon ;
- une composante 12' résultant de la transmission de l'onde lumineuse incidente 12 par l'échantillon, une partie de cette dernière pouvant être absorbée dans l'échantillon.

Ces composantes forment des interférences dans le plan de détection. Aussi, l'image acquise par le capteur d'image comporte des figures d'interférences (ou figures de diffraction), chaque figure d'interférence pouvant être associée à un microorganisme $10_i$ de l'échantillon.

Un processeur 20, par exemple un microprocesseur, est apte à traiter chaque image $I_0$ acquise par le capteur d'image 16. En particulier, le processeur est un microprocesseur relié à une mémoire programmable 22 dans laquelle est stockée une séquence d'instructions pour effectuer les opérations de traitement d'images et de calculs décrites dans cette description. Le processeur peut être couplé à un écran 24 permettant l'affichage d'images acquises par le capteur d'image 16 ou calculées par le processeur 20.

Une image $I_0$ acquise par le capteur d'image 16, également appelée hologramme, ne permet pas d'obtenir une représentation suffisamment précise de l'échantillon observé. Comme décrit en lien avec l'art antérieur, on peut appliquer, à chaque image acquise par le capteur d'image, un opérateur de propagation holographique h, de façon à calculer une grandeur représentative de l'onde lumineuse d'exposition 14. Il est alors possible de reconstruire une expression complexe A de l'onde lumineuse 14 en tout point de coordonnées $(x,y,z)$ de l'espace, et en particulier dans un plan de reconstruction $P_z$ situé à une distance $|z|$ du capteur d'image 16, dite distance de reconstruction, ce plan de reconstruction étant de préférence le plan selon lequel s'étend l'échantillon $P_{10}$, avec :

$A(x,y,z) = I_0(x,y,z) * h$ * désignant l'opérateur produit de convolution, ou, et de préférence,

$$A(x,y,z) = \sqrt{I_0(x,y,z)} * h$$ , ou encore : $$A(x,y,z) = \frac{\sqrt{I_0(x,y,z)}}{\overline{I_0}} * h$$ , $\overline{I_0}$ étant une moyenne de l'image acquise.

L'opérateur de propagation h a pour fonction de décrire la propagation de la lumière entre le capteur d'image 16 et un point de coordonnées (x,y,z), situé à une distance $|z|$ du capteur d'image. Il est alors possible de déterminer le module $M(x,y,z)$ et/ou la phase $\varphi(x,y,z)$ l'onde lumineuse 14, à cette distance $|z|$, dite distance de reconstruction, avec :

- $M(x, y, z) = abs[A(x,y,z)]$ ;
- $\varphi(x, y, z) = arg[A(x, y, z)]$ ;

Les opérateurs *abs* et *arg* désignent respectivement le module et l'argument.

L'opérateur de propagation est par exemple la fonction de Fresnel-Helmholtz, telle que :

$$h(x,y,z) = \frac{1}{j\lambda z} e^{j2\pi\frac{z}{\lambda}} \exp\left(j\pi \frac{x^2+y^2}{\lambda z}\right).$$

Autrement dit, l'expression complexe A de l'onde lumineuse 14, en tout point de coordonnées $(x,y,z)$ de l'espace, est telle que : $A(x, y, z) = M(x, y, z)e^{i\varphi(x,y,z)}$.

Dans la suite de cette description, les coordonnées (x,y) désignent une position radiale dans un plan radial XY perpendiculaire à l'axe de propagation Z. La coordonnée z désigne une coordonnée selon l'axe de propagation Z.

L'expression complexe A est une grandeur complexe dont l'argument et le module sont respectivement représentatifs de la phase et de l'intensité de l'onde lumineuse 14 d'exposition détectée par le capteur d'image 16. Le produit de convolution de l'image $I_0$ par l'opérateur de propagation $h$ permet d'obtenir une image complexe $A_z$ représentant une distribution spatiale de l'expression complexe A dans un plan de reconstruction $P_z$, s'étendant à une distance $|z|$ du plan de détection $P_0$. Dans cet exemple, le plan de détection $P_0$ a pour équation $z = 0$. L'image complexe $A_z$ correspond à une image complexe de l'échantillon dans le plan de reconstruction $P_z$. Elle représente également une distribution spatiale bidimensionnelle des propriétés optiques de l'onde d'exposition 14. Un tel procédé, désigné par le terme reconstruction holographique, permet notamment de reconstruire une image du module ou de la phase de l'onde lumineuse d'exposition 14 dans le plan de reconstruction

Il est possible de former des images $M_z$ et $\varphi_z$ représentant respectivement le module ou la phase d'une image complexe complexe $A_z$ dans un plan $P_z$ situé à une distance $|z|$ du plan de détection $P_0$, avec $M_z = \mathrm{mod}(A_z)$ et $\varphi_z = \arg(A_z)$.

Les inventeurs ont mis au point un procédé de caractérisation de microorganismes, par exemple des levures, ce procédé étant décrit en lien avec la figure 2, et dont certains résultats sont illustrés sur les figures 3A à 3D et 4A à 4C. Ces résultats ont été obtenus selon les conditions expérimentales suivantes :

- Echantillon 10 : il comporte des levures sèches actives ADY (Active Dry Yeast) dispersées dans un milieu de culture Sabouraud, auquel une solution de bleu de méthylène (0.1 mg.ml$^{-1}$) a été ajoutée. La concentration est d'environ 1500 levures par $\mu$l.
- Source de lumière 11 : diode électroluminescente Cree MC-E Color, comportant trois diodes électroluminescentes pouvant être simultanément ou successivement activées, chaque diode émettant respectivement dans les bandes spectrales $\Delta\lambda$ suivantes : 450nm - 465 nm ; 520nm - 535 nm ; 620nm - 630 nm. Dans les essais qui suivent, seule la bande spectrale 620nm - 630 nm a été mise en œuvre.
- Capteur d'image 16 : Capteur 8 bits CMOS monochrome 3884 $\times$ 2764 pixels, chaque pixel mesurant 1.67 $\mu$m de côté, la surface de détection s'étendant sur environ 30 mm$^2$. Compte tenu de l'épaisseur de la chambre fluidique, le volume d'échantillon adressé par chaque image s'élève à 7.5 $\mu$l.
- Distance D entre la source de lumière 11 et l'échantillon 10 : 5 cm.
- Distance d entre l'échantillon 10 et le capteur d'image 16 : 1000 $\mu$m.
- Epaisseur e de la chambre fluidique 15 : 250 $\mu$m.
- Diamètre de l'ouverture du filtre spatial 18 : 150 $\mu$m.

Les principales étapes du procédé de numération selon l'invention sont :

- L'acquisition, par le capteur d'image, d'une image $I_0$ de l'échantillon dans une ou plusieurs bandes spectrales d'illumination, l'image étant acquise à un premier instant $t_1$.
- A partir de l'image acquise, l'obtention d'une première image d'observation $I(t_1)$, correspondant au premier instant, puis, à partir de cette image d'observation, la détection de régions d'intérêt $ROI_i$ correspondant aux microorganismes $10_i$ et la détermination de leurs coordonnées radiales respectives $(x_i, y_i)$, le terme "radial" signifiant dans un plan parallèle au plan de détection.
- A partir de l'image acquise, le calcul d'une grandeur caractéristique, par exemple le module ou la phase, de l'onde lumineuse d'exposition 14, à différentes distances de l'échantillon, dites distances de reconstruction ; cela passe notamment par l'obtention d'une pile d'images complexes représentant l'onde lumineuse d'exposition, les images complexes s'étendant selon des plans parallèles disposés entre le plan de détection $P_0$ et le plan de l'échantillon $P_{10}$.
- La formation d'un profil représentant une évolution de la grandeur caractéristique en fonction de la distance de reconstruction, chaque profil étant associé à un microorganisme.
- A partir d'un ou plusieurs profil associé à chaque microorganisme, l'identification de microorganismes vivants ou morts.

Le procédé peut comporter l'acquisition d'une image de l'échantillon à un deuxième instant $t_2$, le deuxième instant étant postérieur au premier instant $t_1$, et l'obtention d'une deuxième image d'observation $I(t_2)$ associée au deuxième instant. La comparaison des images d'observation respectivement associées au premier et au deuxième instant permet d'identifier, parmi les microorganismes considérés comme vivants, les microorganismes viables non cultivables.

La notion de microorganisme viable et non cultivable (VNC) est connue de l'homme du métier. Il s'agit d'un microorganisme vivant, car présentant une activité métabolique, mais ne pouvant pas se diviser dans le milieu dans lequel il est plongé. Il ne peut donc pas se développer dans son milieu. L'état VNC peut être influencé par différents facteurs, par exemple la température, la teneur de l'environnement ou du milieu de culture en certains nutriments ou éléments chimiques, l'exposition à la lumière, autant de facteurs pouvant induire un stress au microorganisme. L'identification de microorganismes VNC permet une caractérisation rigoureuse de la qualité de l'échantillon considéré.

Etape 100 : Acquisition d'une image $I_0$ de l'échantillon 10 par le capteur d'image 16, cette image formant un hologramme. Un des intérêts de la configuration sans lentille, représentée sur la figure 1, est le large champ observé, permettant d'adresser simultanément un volume d'échantillon élevé. Cela permet d'observer simultanément plusieurs microorganismes, et ainsi d'obtenir une caractérisation rapide de l'échantillon. Le champ observé dépend de la taille du capteur d'image, en étant légèrement inférieur à la surface de détection de ce dernier, du fait de l'espacement entre les pixels du capteur et l'échantillon. Le champ observé est généralement supérieur à 10 mm$^2$, et est typiquement compris entre 10 mm$^2$ et 50 mm$^2$, ce qui est significativement plus élevé qu'avec un microscope. Dans cet exemple, cette image est acquise à un premier instant $t_1$ et peut être notée $I_0(t_1)$.

<u>Etape 110</u> : Formation d'une image de référence. Du fait de l'absence d'optique de formation d'image, l'image acquise $I_0$ peut comporter un grand nombre de figures d'interférence, et peut ne pas être aisément exploitable pour localiser les microorganismes présents dans le champ observé. Ces derniers sont plus facilement identifiables à partir d'une image complexe reconstruite par application d'un opérateur de propagation holographique à l'image acquise. Dans cet exemple, cette image complexe est une image de référence $A_{ref}$, obtenue en effectuant une reconstruction holographique dans un plan de référence $P_{ref}$ à une image obtenue à partir de l'image acquise $I_0$.

Une première solution est d'appliquer l'opérateur de propagation à l'image acquise $I_0$, ou de préférence à la racine carrée de l'image acquise $\sqrt{I_0}$, éventuellement normalisée par la valeur moyenne $\overline{I_0}$ de l'image acquise. L'image de référence $A_{ref}$ est une image complexe comportant des informations de phase et d'amplitude de l'onde lumineuse 14 à laquelle est exposé le capteur d'image 16. Le plan de référence est un plan avantageusement perpendiculaire à l'axe de propagation Z, et/ou parallèle au plan de détection $P_0$. Il s'agit de préférence du plan de l'échantillon $P_{10}$. En effet, c'est généralement dans ce plan que la résolution spatiale d'une image complexe reconstruite est la meilleure, un tel principe étant à la base des algorithmes dits de focalisation numérique.

Cependant, l'image acquise ne comporte pas d'information relative à la phase de l'onde d'exposition 14. De ce fait, la reconstruction holographique s'effectue sur la base d'une information optique incomplète, basée uniquement sur l'intensité de l'onde lumineuse collectée sur le capteur d'image. L'amélioration de la qualité de la reconstruction holographique a fait l'objet de nombreux développements, en mettant en œuvre des algorithmes fréquemment dénommés « Phase retrieval », permettant une estimation de la phase de l'onde lumineuse à laquelle le capteur d'image est exposé. Ce type d'algorithme permet de limiter le bruit de reconstruction affectant l'image complexe reconstruite $A_{ref}$. Un exemple d'algorithme utilisable est par exemple décrit dans US2012/0218379.

Selon une possibilité, l'échantillon est illuminé successivement ou simultanément dans différentes bandes spectrales $\Delta\lambda_k$, et on acquiert, dans le plan de détection $P_0$, une image $I_0(\Delta\lambda_k)$ représentative de chaque bande spectrale. L'algorithme permet d'obtenir une image complexe $A_{ref}(\Delta\lambda_k)$ de l'échantillon 10, dans le plan de référence, dans chaque bande spectrale $\Delta\lambda_k$. Les images complexes ainsi obtenues peuvent être combinées, par exemple en effectuant une moyenne, en chaque pixel, de leur module et de leur phase, ce qui permet de former l'image de référence $A_{ref}$. Alternativement, l'image complexe de référence est une image complexe $A_{ref}(\Delta\lambda_k)$ dans une bande spectrale $\Delta\lambda_k$. Un tel algorithme a été décrit dans la publication S. N. A. Morel, A. Delon, P. Blandin, T. Bordy, O. Cioni, L. Hervé, C. Fromentin, J. Dinten, and C. Allier, "Wide-Field Lensfree Imaging of Tissue Slides," in Advanced Microscopy Techniques IV*; and* Neurophotonics II, E. Beaurepaire, P. So, F. Pavone, and E. Hillman, eds., Vol. 9536 of SPIE Proceedings (Optical Society of America, 2015) ainsi que dans la demande de brevet FR1554811 déposée le 28 mai 2015, et plus précisément dans les étapes itératives 100 à 500 décrites dans cette demande. On a montré que l'utilisation de deux ou trois bandes spectrales différentes permet d'obtenir une bonne qualité de reconstruction.

Une autre possibilité, qui correspond au mode de réalisation préféré, est de reconstruire une image complexe de référence en se basant sur une image acquise de l'échantillon lorsque ce dernier est illuminé dans une seule bande spectrale $\Delta\lambda$. L'image complexe de référence peut être obtenue en utilisant un algorithme itératif tel que décrit dans la demande de brevet FR1652500 déposée le 23 mars 2016, et plus précisément selon les étapes 110 à 160 décrites dans ladite demande de brevet.

La coordonnée $z_{ref}$ du plan de référence $P_{ref}$ est déterminée soit a priori, notamment lorsque la position de l'échantillon est maîtrisée par rapport au capteur d'image 16, soit par le biais d'une focalisation numérique. La focalisation numérique permet de définir un plan de focalisation $P_{focus}$ reconstruisant plusieurs images et définissant un critère de netteté de chaque image reconstruite. Le plan de focalisation $P_{focus}$ correspond à celui dans lequel l'image reconstruite présente un critère de netteté optimal. Il correspond au plan dans lequel s'étend une majorité des microorganismes. L'image de référence $A_{ref}$ est alors formée dans un plan de référence $P_{ref}$ correspondant au plan de focalisation $P_{focus}$. Le plan de focalisation correspond à un plan selon lequel s'étend l'échantillon.

L'image complexe $A_{ref}$ est désignée comme étant une image de référence, car elle sert de base à la formation de profils sur la base duquel les microorganismes de l'échantillon sont caractérisés. La figure 3A montre une image du module $M_{ref}$ de l'image complexe de référence obtenue en mettant en œuvre l'algorithme décrit dans le paragraphe précédent.

<u>Etape 120</u> : construction d'une pile d'images.

Cette étape comporte une application d'un l'opérateur de propagation h à l'image complexe de référence $A_{ref}$ de façon à calculer des images complexes $A_{ref,z}$, dites secondaires, le long de l'axe de propagation Z. Au cours de cette étape, l'image complexe de référence $A_{ref}$ est propagée selon une pluralité de distances de reconstruction z, en utilisant un opérateur de propagation h tel que précédemment défini, de façon à disposer d'une pluralité d'images complexes, dites secondaires, $A_{ref,z}$ reconstruites aux différentes distances z du plan de référence $P_{ref}$. Ainsi, cette étape comprend la détermination d'une pluralité d'images complexes $A_{ref,z}$ telles que :

$$A_{ref,z} = A_{ref} * h_z \text{ avec } z_{min} \leq z \leq z_{max}.$$

On obtient ainsi une pile d'images complexes $A_{ref,z_{min}}$... $A_{ref,z_{max}}$.

Les valeurs $z_{min}$ et $z_{max}$ sont les coordonnées minimales et maximales, selon l'axe Z, entre lesquelles l'image complexe de référence est propagée. De préférence, les images complexes sont reconstruites selon une pluralité de coordonnées z entre l'échantillon 10 et le capteur d'image 16. Les inventeurs ont estimé qu'il était préférable d'obtenir des images complexes secondaires de part et d'autre du plan de référence $P_{ref}$, de telle sorte que $z_{min} \leq z_{ref} \leq z_{max}$. Contrairement à l'image acquise $I_0$ par le capteur d'image 16, l'image complexe de référence décrit correctement l'onde lumineuse d'exposition 14, en particulier au niveau de sa phase. Par conséquent, on estime que les images secondaires $A_{ref,z}$, obtenues par propagation de l'image de référence, forment un bon descripteur de la propagation de l'onde lumineuse d'exposition 14 selon l'axe de propagation Z. Ainsi, les images complexes secondaires sont calculées rapidement, sans nécessiter la mise en œuvre d'un procédé itératif tel que celui mis en œuvre pour calculer l'image complexe de référence $A_{ref}$. Le procédé consistant à appliquer un algorithme itératif pour établir une image complexe de référence $A_{ref}$ (étape 110) puis, d'obtenir des images complexes secondaires par application d'un opérateur de propagation h à l'image complexe de référence, permet d'obtenir une pile d'images complexes $A_{ref,z}$ en optimisant les moyens de calcul.

De préférence, deux plans de reconstructions adjacents sont espacés les uns des autres selon un maillage fin, compris par exemple entre 5 μm et 50 μm, et par exemple 25 μm. Il s'agit d'une propagation locale, car réalisée selon une distance comprise entre 500 μm et 2 μm de part et d'autre du plan de référence $P_{ref}$, par exemple à ± 500 μm. En se basant sur une reconstruction selon une distance de 500 μm de part et d'autre du plan de référence $P_{ref}$ et une distance entre deux plans adjacents de 20 μm, on propage l'image complexe de référence $A_{ref}$ selon quarante plans de reconstruction $P_{ref,z}$, de façon à former autant d'images complexes secondaires, $A_{ref,z}$.

De façon alternative, les étapes 110 et 120 peuvent être remplacées par une propagation à partir de l'image $I_0$ acquise lors de l'étape 100, selon l'étape 110' représentée sur la figure 2. La propagation peut être effectuée par exemple en appliquant un opérateur de propagation à la racine carrée $\sqrt{I_0}$ de cette image, éventuellement normalisée par la valeur moyenne $\overline{I_0}$ selon différentes distances de propagation $z_1$ ....$z_n$. Il peut s'agir d'une simple application d'un opérateur de propagation $h$, auquel cas les images reconstruites $A_{z_1}$...$A_{z_n}$, formant la pile d'images complexes, peuvent être affectées d'un bruit de reconstruction important. Il peut également s'agir d'une mise en œuvre d'un algorithme de reconstruction holographique itératif, par exemple un des algorithmes précédemment évoqués, selon différentes distances de propagation $z_1$ ....$z_n$. Cela permet d'obtenir des images complexes reconstruites de bonne qualité, mais le procédé est plus coûteux en tant de calcul. C'est pourquoi il est préférable de mettre en œuvre un algorithme de reconstruction itératif pour former l'image complexe de référence, selon l'étape 110, et de propager l'image complexe de référence par une simple application d'un opérateur de propagation, selon l'étape 120. Cela permet d'obtenir un bon compromis entre la qualité des images formant la pile d'images complexes et le temps de calcul.

Etape 130 : détection de microorganismes dans l'échantillon.

Cette étape vise à détecter chaque microorganisme présent dans l'échantillon. Elle est effectuée à partir d'une image d'observation $I(t_1)$, dite première image d'observation, formée à partir d'une l'image $I_0(t_1)$ acquise par le capteur d'image à un premier instant d'acquisition $t_1$, ou à partir d'une image complexe formée à partir de cette dernière. De préférence, sans pour autant que cela soit nécessaire, l'image $I_0(t_1)$ correspond à l'image $I_0$ acquise lors de l'étape 100 et la première image d'observation $I(t_1)$ résulte de cette image acquise $I_0$, ou d'une image complexe de la pile d'images formée lors de l'étape 120. Il est préférable que l'image d'observation $I(t_1)$ soit établie à partir du module et/ou de la phase d'une image complexe reconstruite dans un plan selon lequel s'étend l'échantillon. Il peut par exemple s'agir de l'image complexe de référence $A_{ref}$ obtenue lors de l'étape 110 ou d'une image complexe secondaire $A_{ret,z}$ résultant de l'étape 120. La première image d'observation $I(_1)$ peut être l'image du module $M_{ref}$ de l'image complexe de référence $A_{ref}$ ou l'image $M_{ref,z}$ du module d'une autre image complexe de la pile d'images formée lors de l'étape 120. Il peut également s'agir de l'image $\varphi_{ref}$ de la phase de l'image complexe de référence $A_{ref}$, ou de l'image $\varphi_{ref,z}$ de la phase d'une autre image complexe de la pile d'images formée lors de l'étape 120. D'une façon générale, l'image d'observation est obtenue à partir du module et/ou de la phase d'une image complexe résultant d'une propagation d'une image $I_0$ acquise par le capteur d'image 16.

La détection de chaque microorganisme est effectuée soit manuellement par un opérateur, soit automatiquement, par une analyse morphologique sur la première image d'observation $I(t_1)$, en prenant en compte le fait que chaque micro-organisme $10_i$ peut être associé, dans l'image d'observation, à une région d'intérêt $ROI_i$ de forme prédéterminée, pouvant être aisément détectée. Il peut par exemple s'agir d'une forme circulaire ou ellipsoïdale, ou autre. Pour cela, le procédé peut détecter automatiquement chaque région d'intérêt $ROI_i$ en prenant compte un ou plusieurs critères morphologiques correspondant à un microorganisme $10_j$, par exemple son aire et son excentricité. A chaque région d'intérêt $ROI_i$ détectée correspond au moins un microorganisme $10_j$. Des algorithmes basés sur une corrélation spatiale avec des formes de régions d'intérêt prédéterminées peuvent également être mis en œuvre. Le volume de l'échantillon 10 dans le champ d'observation du capteur d'image 16 étant connu, cette étape permet une estimation d'une quantité $N_i$ ou d'une concentration de microorganismes $10_i$ dans l'échantillon.

La figure 3A correspond à une image du module $M_{ref}$ d'une image complexe de référence d'un échantillon décrit ci-après, en lien avec les exemples. La figure 3B correspond à un détail de la figure 3A.

Etape 140 : détection des coordonnées radiales $(x_i, y_i)$ de chaque microorganisme.

Les microorganismes $10_i$ étant détectés, par l'intermédiaire des régions d'intérêt $ROI_i$ qui leur sont respectivement associées sur la première image d'observation $1(t_1)$, leur position $(x_i, y_i)$ dans le plan radial XY, c'est-à-dire dans un plan parallèle au plan de détection, peut être déterminée aisément, en considérant par exemple le centroïde de la région d'intérêt $ROI_i$ correspondant à chacun d'entre eux.

Etape 150 : formation d'un profil associé à chaque microorganisme.

A partir de chaque image complexe formant la pile d'images, on estime une grandeur caractéristique de l'onde lumineuse d'exposition 14, à chaque position radiale $(x_i, y_i)$ sélectionnée lors de l'étape 140, et à une pluralité de distances z de reconstruction du plan de référence $P_{ref}$, ou du plan de détection $P_0$, puis on forme un profil représentant une évolution de la grandeur caractéristique en fonction de z, selon l'axe de propagation Z. La grandeur caractéristique peut notamment être établie à partir du module et de la phase de l'expression complexe A décrivant l'onde lumineuse d'exposition 14, en utilisant les images de la pile d'images complexes préalablement résultant des étapes 120 ou 110'.

Les figures 3C et 3D représentent respectivement un profil $M_i(z)$ du module et un profil $\varphi_i(z)$ de la phase, passant par les positions radiales $(x_i, y_i)$ sélectionnées sur l'image 3B. Chaque profil est obtenu à partir des images de la pile d'images complexes préalablement établie, en effectuant une interpolation entre les coordonnées de deux images reconstruites adjacentes. Chaque profil est associé à un micro-organisme $10_i$.

Etape160 : classification de chaque microorganisme à partir des profils formés au cours de l'étape 150.

Des essais ont montré que lorsqu'un marqueur de viabilité est préalablement introduit dans l'échantillon, les profils $M_i(z)$ décrivant l'évolution du module de l'onde lumineuse d'exposition 14 peuvent permettre une classification entre les microorganismes morts $10_{i,d}$ ou les microorganismes vivants $10_{i,a}$. Ces profils sont représentés sur la figure 3C. Sur cette figure, la coordonnée z = 20 correspond au plan de focalisation, c'est-à-dire au plan selon lequel s'étendent les microorganismes. Un critère de classification est par exemple la valeur maximale prise par chaque profil $M_i(z)$ entre le plan de détection et le plan de focalisation. Sur la figure 3C, il s'agit de la valeur maximale sur une partie de chaque profil $M_i(z)$ s'étendant entre les coordonnées z = 0 et z = 20. Lorsque la valeur maximale d'un profil est inférieure à un seuil prédéterminé, le microorganisme est classé comme étant mort. Dans le cas contraire, le microorganisme est classé comme étant vivant. Sur les figures 3C et 3D, les profils correspondant aux microorganismes considérés comme morts et vivants sont représentés respectivement en pointillés (légende "d") et en tirets (légende "a").

Le procédé comporte de préférence les étapes 170 et 180 visant à identifier, parmi les microorganismes considérés comme vivants à l'issue de l'étape 160, les microorganismes viables non cultivables (VNC) précédemment évoqués.

Etape 170 : acquisition d'une image différée $I_0(t_2)$.

Au cours de l'étape 130, une première image d'observation $I(t_1)$ a été formée, représentant l'échantillon à un premier instant, ce premier instant étant noté $t_1$. L'étape 170 comporte une acquisition, à un deuxième instant d'acquisition $t_2$, postérieur au premier instant d'acquisition $t_1$, d'une deuxième image $I_0(t_2)$, dite image différée, de l'échantillon 10 à l'aide du capteur d'image 16. Les inventeurs ont observé qu'il était préférable que l'intervalle temporel $\Delta t$ entre le premier instant $t_1$ et le deuxième instant $t_2$ soit supérieur à 4h, et de préférence supérieur à 15h ou 20h. Par exemple l'intervalle temporel $\Delta t$ est compris entre 4 et 72 heures, cet intervalle étant ajusté en fonction de la durée nécessaire à une division du microorganisme considéré.

Etape 180 : formation d'une image d'observation différée et classification des microorganismes vivants.

Sur la base de l'image acquise au deuxième temps d'acquisition, une deuxième image d'observation $I(t_2)$ est formée. La deuxième image d'observation $I(t_2)$ est de préférence formée de la même façon que la première image d'observation $I(t_1)$, de façon que les microorganismes soient comparables sur ces deux images. A partir de la deuxième image d'observation, on observe les microorganismes $10_{i,a}$ considérés comme vivants suite à l'étape 160. Parmi ces derniers, on identifie les microorganismes dont la morphologie a varié par rapport à celle observée sur la première image d'observation $I(t_1)$. La variation de la morphologie, obtenue par comparaison entre la première et la deuxième image d'observation, doit traduire au moins une division du microorganisme durant l'intervalle temporel $\Delta t$. Elle peut être déterminée manuellement ou automatiquement, par exemple sur la base d'une comparaison, entre les deux images d'observation $I(t_1)$ et $I(t_2)$, de l'aire ou d'un paramètre de forme de la région d'intérêt $ROI_i$ associée, sur chacune de ces images, à chaque microorganisme vivant. Par exemple, lorsque l'aire d'une région d'intérêt $ROI_i$ a augmenté de plus de 20% entre les deux images, on considère que le microorganisme $10_{i,a}$ s'est divisé ou qu'une division est en cours. Dans ce cas, il est considéré comme vivant et apte à ce diviser, donc cultivable. Dans le cas contraire, il est considéré comme viable non cultivable.

Les figures 4B et 4C représentent respectivement :

- la première image d'observation $I(t_1)$, décrite en lien avec l'étape 110, qui correspond au module $M_{ref}$ de l'image complexe de référence $A_{ref}$ ;
- une deuxième image d'observation $I(t_2)$, obtenue à partir d'une image $I_0(t_2)$ acquise à un deuxième instant $t_2$ postérieur de 10 heures au premier instant $t_1$. La deuxième image d'observation est le module d'une image complexe obtenue, à partir de l'image $I_0(t_2)$, de la même façon que l'image complexe de référence $A_{ref}$.

Sur la figure 4B, les microorganismes $10_{i,a}$ considérés comme vivants sont contournés d'un cadre blanc, tandis que les microorganismes $10_{i,d}$ considérés comme morts sont contournés par un cercle blanc. A la position radiale ($x_i$, $y_i$) correspondant à un microorganisme $10_{i,a}$ considéré comme vivant, une comparaison est effectuée entre la région d'intérêt $ROI_i$ s'étendant autour de la position radiale aux premier et deuxième instants. Sur la base de cette comparaison, les microorganismes $10_{i,a1}$ considérés comme vivants et cultivables sont entourés par un cadre plein sur la figure 4C tandis que les microorganismes $10_{i,a2}$ considérés comme viables non cultivables sont entourés par un cadre en pointillés.

L'invention permet alors un dénombrement de trois catégories de microorganismes : morts, vivants et cultivables, viables non cultivables.

Les inventeurs ont constaté que la performance du procédé était accrue lorsque la bande spectrale d'illumination $\Delta\lambda$ est différente de la bande spectrale de coloration $\Delta\lambda'$ induite par le marqueur de viabilité. Ainsi, lorsque le marqueur de viabilité est du bleu de méthylène, la bande spectrale d'illumination est de préférence située dans le rouge ou dans l'infrarouge. Ainsi, il est préférable que la bande spectrale d'illumination et la bande spectrale de coloration ne se recoupent pas, ou se recoupent de façon marginale. Par se recouper de façon marginale, on entend que l'intersection entre les deux bandes spectrales est inférieur à 10% ou 20% d'une des deux bandes spectrales.

Le procédé décrit ci-avant a été comparé avec une caractérisation visuelle des microorganismes, ces derniers étant observés au microscope par un opérateur. La figure 4A représente une vue au microscope au deuxième instant $t_2$. La caractérisation visuelle a été utilisée en tant que méthode de référence, pour qualifier le procédé selon l'invention.

Le tableau suivant montre les pourcentages de microorganismes considérés comme morts, vivants et cultivables, viables non cultivables, obtenus respectivement selon la méthode décrite ci-dessus (première ligne) et au microscope (2$^{\text{ième}}$ ligne).

|  | morts | vivants cultivables | viables non cultivables |
|---|---|---|---|
| algorithme | 15 | 34 | 51 |
| référence | 12 | 24 | 64 |

La proportion élevée de levures viables non cultivables s'explique par une température élevée à laquelle a été porté l'échantillon, engendrant un stress favorisant l'état viable non cultivable.

La fiabilité du procédé est donc comparable à une caractérisation visuelle classique réalisée au microscope. Mais du fait de la configuration en imagerie sans lentille, le champ observé est nettement plus important que le champ observé résultant de l'utilisation d'un microscope. L'invention a par exemple permis une caractérisation simultanée de 11574 levures réparties dans le champ d'observation. La performance de l'invention est donc supérieure au procédé visuel en termes de quantité de microorganismes caractérisés par unité de temps. De plus, l'algorithme peut être automatisé, la fiabilité étant démontrée par les valeurs du tableau précédent.

Selon une variante, une optique de formation d'image est disposée entre l'échantillon et le capteur d'image, le capteur d'image étant localisé selon une configuration dite défocalisée, le plan focal objet de l'optique étant décalé du plan selon lequel s'étend l'échantillon selon une distance dite de défocalisation. La distance de défocalisation peut être comprise entre 5 $\mu$m et 5 mm, et de préférence entre 10 $\mu$m et 2 mm. De la même manière qu'en configuration sans lentille, une telle configuration permet l'obtention d'une image dans laquelle chaque microorganisme apparaît sous la forme d'une figure de diffraction, des interférences se produisant entre l'onde lumineuse émise par la source de lumière et se propageant jusqu'au capteur d'image et une onde de diffraction générée par chaque microorganisme. Le procédé décrit en lien avec les étapes 100 à 180 est applicable à des images acquises selon une telle configuration. Toutefois, une configuration en imagerie sans lentille est préférée, par le plus grand champ d'observation qu'elle procure.

Bien que décrite relativement à une caractérisation de levures, à des fins de contrôle de qualité, l'invention s'applique à d'autres organismes tels que ceux précédemment listés, dès lors que l'on souhaite obtenir une analyse rapide et fiable dans un champ d'observation important.

## Revendications

1.  Procédé d'analyse de microorganismes ($10_i$), les microorganismes étant disposés dans un échantillon, l'échantillon comportant un marqueur de viabilité ($10_v$), apte à modifier une propriété optique des microorganismes de façon différente selon qu'ils sont morts ou vivants, le procédé comportant les étapes suivantes :

    a) illumination de l'échantillon à l'aide d'une source de lumière (11), la source de lumière émettant une onde lumineuse incidente (12) se propageant vers l'échantillon (10) selon un axe de propagation (Z) ;
    b) acquisition, à l'aide d'un capteur d'image (16), d'une image ($I_0$) de l'échantillon (10), formée dans un plan de détection ($P_0$), l'échantillon étant disposé entre la source de lumière (11) et le capteur d'image (16), l'image étant représentative d'une onde lumineuse (14) d'exposition, à laquelle est exposé le capteur d'image (16) sous l'effet de l'illumination, l'image comportant des figures d'interférences entre une partie (12') de l'onde lumineuse incidente (12) transmise par l'échantillon et la diffraction de l'onde incidente (12) par les microorganismes ;

    le procédé étant **caractérisé en ce qu'**il comprend également les étapes suivantes :

    c) détermination de positions radiales ($x_i$, $y_i$) de différents microorganismes ($10_i$) dans un plan parallèle au plan de détection, chaque position radiale étant associée à un microorganisme ;
    d) à partir de l'image acquise ($I_0$) lors de l'étape b), application d'un opérateur de propagation *(h),* de façon à calculer au moins une grandeur caractéristique ($M$, $\varphi$) de l'onde lumineuse d'exposition (14), à chaque position radiale ($x_i$, $y_i$) déterminée lors de l'étape c), et à une pluralité de distances (z) du plan de détection ($P_0$);
    e) formation d'un profil ($M_i(z)$, $\varphi_i(z)$), représentant une évolution de la grandeur caractéristique calculée lors de l'étape d) selon un axe parallèle à l'axe de propagation (Z) et passant par chaque position radiale ($x_i$, $y_i$) déterminée lors de l'étape c), chaque profil étant associé à un microorganisme ($10_i$);
    f) en fonction de chaque profil formé lors de l'étape e), classification entre les microorganismes morts et vivants.

2.  Procédé selon la revendication 1, comportant, suite à l'étape f), une étape g) d'analyse de la faculté du microorganisme à se diviser, l'étape g) comportant les sous-étapes suivantes pour au moins un microorganisme considéré comme vivant lors de l'étape f) :

    gi) obtention d'une première image d'observation ($I(t_1)$) de l'échantillon, à un premier instant ($t_1$), l'image d'observation comportant des régions d'intérêt ($ROI_i$) respectivement associées à des microorganismes ($10_i$) et détection d'une région d'intérêt ($ROI_i$) associée audit microorganisme ($10_{i,a}$);
    gii) acquisition d'une image de l'échantillon ($I_0(t_2)$) à un deuxième instant ($t_2$), le deuxième instant étant postérieur au premier instant, et obtention d'une deuxième image d'observation ($I(t_2)$) de l'échantillon à partir de l'image ($I_0(t_2)$) de l'échantillon acquise au deuxième instant ;
    giii) détection, sur la deuxième image d'observation, d'une région d'intérêt ($ROI_j$) correspondant au microorganisme ($10_{i,a}$);
    giv) comparaison des régions d'intérêt détectées lors des sous-étapes gi) et giii) ;
    gv) détermination de la faculté du microorganisme à se diviser en fonction de la comparaison effectuée lors de la sous-étape giv).

3.  Procédé selon la revendication 2, dans lequel la sous-étape gv) comporte l'identification, parmi les microorganismes vivants ($10_{i,a}$), de microorganismes viables et non cultivables ($10_{i,a2}$).

4.  Procédé selon la revendication 2 ou la revendication 3, dans lequel lors des sous-étapes gi) et gii), la première image d'observation ($I(t_1)$) et la deuxième image d'observation ($I(t_2)$) sont obtenues par application d'un opérateur de propagation (h) respectivement à partir d'une image ($I_0(t_1)$) acquise au premier instant et à partir de l'image

$(I_0(t_2))$ acquise au deuxième instant.

5. Procédé selon l'une quelconque des revendications 2 à 4 dans lequel lors de la sous-étape gi), la première image d'observation ($I(t_1)$) est obtenue à partir de l'image acquise ($I_0$) lors de l'étape b).

6. Procédé selon l'une quelconque des revendications 2 à 5, dans lequel l'intervalle temporel ($\Delta t$) entre le premier instant et le deuxième instant est compris entre 5 heures et 70 heures.

7. Procédé selon l'une quelconque des revendications précédentes dans lequel lors de l'étape d) l'opérateur de propagation est appliqué à partir de l'image acquise ($I_0$) lors de l'étape b), selon une pluralité de distances de propagation, de façon à obtenir une pile d'images complexes.

8. Procédé selon l'une quelconque des revendications 1 à 6 dans lequel l'étape d) comporte : les sous-étapes suivantes :

   di) application d'un opérateur de propagation (h), à partir de l'image ($I_0$) acquise lors de l'étape b) de façon à calculer une image complexe ($A_{ref}$), dite image de référence, représentative de l'échantillon, dans un plan de référence ($P_{ref}$) ;
   dii) application d'un opérateur de propagation (h) à l'image de référence ($A_{ref}$), de façon à obtenir des images complexes, dites images complexes secondaires ($A_{ref,z}$), à différentes distances (z) du plan de référence ($P_{ref}$) selon l'axe de propagation (Z), les images complexes secondaires et l'image de référence formant une pile d'images complexes ;
   diii) détermination d'une position radiale ($x_i, y_i$) de microorganismes ($10_i$) à partir des images de la pile d'images complexes obtenue lors de la sous-étape dii).

9. Procédé selon l'une quelconque des revendications précédentes, dans lequel le marqueur de viabilité ($10_v$) induit une coloration des microorganismes lorsqu'ils sont morts, selon une bande spectrale de coloration ($\Delta\lambda'$), et dans lequel lors de l'étape a), l'illumination de l'échantillon est effectuée selon une bande spectrale d'illumination ($\Delta\lambda$), la bande spectrale d'illumination ne comportant pas tout ou partie de la bande spectrale de coloration.

10. Procédé selon l'une quelconque des revendications précédentes, dans lequel dans l'étape d), la grandeur caractéristique est déterminée à partir du module ou de la phase de l'onde lumineuse d'exposition, à chaque distance du plan de détection.

11. Procédé selon l'une quelconque des revendications précédentes, dans lequel l'étape f) comporte une classification de chaque profil ($M_i(z)$, $\varphi_i(z)$) selon une première classe, correspondant à des profils caractéristiques de microorganismes vivants ($10_{i,a}$) et une deuxième classe, correspondant à des profils caractéristiques de microorganismes morts ($10_{i,d}$).

12. Procédé selon la revendication 11 dans lequel la classification de chaque profil ($M_i(z)$, $\varphi_i(z)$) est effectuée en fonction de sa forme ou d'une valeur maximale ou d'une valeur minimale du profil.

13. Procédé selon l'une quelconque des revendications précédentes, dans lequel aucune optique de formation d'image n'est disposée entre l'échantillon et le capteur d'image.

14. Procédé selon l'une quelconque des revendications 1 à 12, dans lequel un système optique de formation d'image est disposé entre l'échantillon et le capteur d'image, le système optique présentant un plan focal objet, l'échantillon s'étendant dans un plan de l'échantillon, le plan de l'échantillon étant décalé par rapport au plan focal objet.

**Patentansprüche**

1. Verfahren zur Analyse von Mikroorganismen ($10_i$), wobei die Mikroorganismen in einer Probe angeordnet sind, wobei die Probe einen Lebensfähigkeitsmarker ($10_v$) aufweist, der eine optische Eigenschaft der Mikroorganismen unterschiedlich ändern kann, je nachdem, ob sie tot oder lebendig sind, wobei das Verfahren die folgenden Schritte aufweist:

   a) Beleuchtung der Probe mit Hilfe einer Lichtquelle (11), wobei die Lichtquelle eine einfallende Lichtwelle (12) emittiert, die sich gemäß einer Ausbreitungsachse (Z) zur Probe (10) hin ausbreitet;

b) Gewinnung, mit Hilfe eines Bildsensors (16), eines in einer Erfassungsebene ($P_0$) geformten Bilds ($I_0$) der Probe (10), wobei die Probe zwischen der Lichtquelle (11) und dem Bildsensor (16) angeordnet ist, wobei das Bild für eine Belichtungslichtwelle (14) repräsentativ ist, der der Bildsensor (16) unter der Wirkung der Beleuchtung ausgesetzt wird, wobei das Bild Interferenzfiguren zwischen einem Teil (12') der von der Probe übertragenen einfallenden Lichtwelle (12) und der Beugung der einfallenden Welle (12) durch die Mikroorganismen aufweist;

wobei das Verfahren **dadurch gekennzeichnet ist, dass** es ebenfalls die folgenden Schritte enthält:

c) Bestimmung radialer Positionen ($x_i$, $y_i$) verschiedener Mikroorganismen ($10_i$) in einer Ebene parallel zur Erfassungsebene, wobei jede radiale Position einem Mikroorganismus zugeordnet ist;

d) ausgehend vom im Schritt b) gewonnenen Bild ($I_0$), Anwendung eines Ausbreitungsoperators *(h),* um mindestens eine charakteristische Größe *(M, <p)* der Belichtungslichtwelle (14) in jeder im Schritt c) bestimmten radialen Position ($x_i$, $y_i$) und in einer Vielzahl von Abständen (z) der Erfassungsebene ($P_0$) zu berechnen;

e) Formen eines Profils ($M_i(z)$, $\varphi_i(z)$), das eine Entwicklung der im Schritt d) berechneten charakteristischen Größe gemäß einer Achse parallel zur Ausbreitungsachse (Z) und durch jede im Schritt c) bestimmte radiale Position ($x_i$, $y_i$) verlaufend darstellt, wobei jedes Profil einem Mikroorganismus ($10_i$) zugeordnet ist;

f) abhängig von jedem im Schritt e) geformten Profil, Klassifizierung zwischen den toten und lebenden Mikroorganismen.

2. Verfahren nach Anspruch 1, das nach dem Schritt f) einen Schritt g) der Analyse der Fähigkeit des Mikroorganismus, sich zu teilen, aufweist, wobei der Schritt g) die folgenden Teilschritte für mindestens einen Mikroorganismus aufweist, der im Schritt f) als lebend angesehen wird:

gi) Erhalt eines ersten Beobachtungsbilds ($I(t_1)$) der Probe zu einem ersten Zeitpunkt ($t_1$), wobei das Beobachtungsbild interessierende Bereiche ($ROI_i$) aufweist, die je Mikroorganismus ($10_i$) zugeordnet sind, und Erfassung eines dem Mikroorganismus ($10_{i,a}$) zugeordneten interessierenden Bereichs ($ROI_i$);

gii) Gewinnung eines Bilds der Probe ($I_0(t_2)$) zu einem zweiten Zeitpunkt ($t_2$), wobei der zweite Zeitpunkt nach dem ersten Zeitpunkt liegt, und Erhalt eines zweiten Beobachtungsbilds ($I(t_2)$) der Probe ausgehend vom zum zweiten Zeitpunkt gewonnenen Bild ($I_0(t_2)$) der Probe;

giii) Erfassung, auf dem zweiten Beobachtungsbild, eines interessierenden Bereichs ($ROI_i$) entsprechend dem Mikroorganismus ($10_{i,a}$);

giv) Vergleich der in den Teilschritten gi) und giii) erfassten interessierenden Bereiche;

gv) Bestimmung der Fähigkeit des Mikroorganismus, sich zu teilen, abhängig vom im Teilschritt giv) ausgeführten Vergleich.

3. Verfahren nach Anspruch 2, wobei der Teilschritt gv) die Identifizierung unter den lebenden Mikroorganismen ($10_{i,a}$) von lebensfähigen und nicht kultivierbaren Mikroorganismen ($10_{i,a2}$) aufweist.

4. Verfahren nach Anspruch 2 oder Anspruch 3, wobei in den Teilschritten gi) und gii) das erste Beobachtungsbild ($I(t_1)$) und das zweite Beobachtungsbild ($I(t_2)$) durch Anwenden eines Ausbreitungsoperators (*h*) ausgehend von einem zum ersten Zeitpunkt gewonnenen Bild ($I_0(t_1)$) bzw. ausgehend von dem zum zweiten Zeitpunkt gewonnenen Bild ($I_0(t_2)$) erhalten werden.

5. Verfahren nach einem der Ansprüche 2 bis 4, wobei im Teilschritt gi) das erste Beobachtungsbild ($I(t_1)$) ausgehend vom im Schritt b) gewonnenen Bild ($I_0$) erhalten wird.

6. Verfahren nach einem der Ansprüche 2 bis 5, wobei das Zeitintervall ($\Delta t$) zwischen dem ersten Zeitpunkt und dem zweiten Zeitpunkt zwischen 5 Stunden und 70 Stunden liegt.

7. Verfahren nach einem der vorhergehenden Ansprüche, wobei im Schritt d) der Ausbreitungsoperator ausgehend vom im Schritt b) gewonnenen Bild ($I_0$) gemäß einer Vielzahl von Ausbreitungsabständen angewendet wird, um einen Stapel komplexer Bilder zu erhalten.

8. Verfahren nach einem der Ansprüche 1 bis 6, wobei der Schritt d) die folgenden Teilschritte aufweist:

di) Anwendung eines Ausbreitungsoperators (h) ausgehend vom im Schritt b) gewonnenen Bild ($I_0$), um ein für

die Probe repräsentatives komplexes Bild ($A_{ref}$), Bezugsbild genannt, in einer Bezugsebene ($P_{ref}$) zu berechnen;

dii) Anwendung eines Ausbreitungsoperators *(h)* an das Bezugsbild ($A_{ref}$), um komplexe Bilder, sekundäre komplexe Bilder genannt ($A_{ref,z}$), in unterschiedlichen Abständen (z) von der Bezugsebene ($P_{ref}$) gemäß der Ausbreitungsachse (Z) zu erhalten, wobei die sekundären komplexen Bilder und das Bezugsbild einen Stapel komplexer Bilder formen;

diii) Bestimmen einer radialen Position ($x_i$, $y_i$) von Mikroorganismen ($10_i$) ausgehend von den Bildern des im Teilschritt dii) erhaltenen Stapels komplexer Bilder.

9. Verfahren nach einem der vorhergehenden Ansprüche, wobei der Lebensfähigkeitsmarker ($10_v$) eine Färbung der Mikroorganismen, wenn sie tot sind, gemäß einem Färbungsspektralband ($\Delta\lambda$') induziert, und wobei im Schritt a) die Beleuchtung der Probe gemäß einem Beleuchtungsspektralband ($\Delta\lambda$) ausgeführt wird, wobei das Beleuchtungsspektralband das Färbungsspektralband nicht ganz oder teilweise aufweist.

10. Verfahren nach einem der vorhergehenden Ansprüche, wobei im Schritt d) die charakteristische Größe ausgehend vom Modul oder von der Phase der Belichtungslichtwelle in jedem Abstand von der Erfassungsebene bestimmt wird.

11. Verfahren nach einem der vorhergehenden Ansprüche, wobei der Schritt f) eine Klassifizierung jedes Profils ($M_i(z)$, $\varphi_i(z)$) gemäß einer ersten Klasse, die charakteristischen Profilen lebender Mikroorganismen ($10_{i,a}$) entspricht, und einer zweiten Klasse aufweist, die charakteristischen Profilen toter Mikroorganismen ($10_{i,d}$) entspricht.

12. Verfahren nach Anspruch 11, wobei die Klassifizierung jedes Profils ($M_i(z)$, $\varphi_i(z)$) abhängig von seiner Form oder von einem maximalen Wert oder einem minimalen Wert des Profils ausgeführt wird.

13. Verfahren nach einem der vorhergehenden Ansprüche, wobei keine Bildformungsoptik zwischen der Probe und dem Bildsensor angeordnet ist.

14. Verfahren nach einem der Ansprüche 1 bis 12, wobei ein optisches Bildformungssystem zwischen der Probe und dem Bildsensor angeordnet ist, wobei das optische System eine Objektbrennebene aufweist, wobei die Probe sich in einer Ebene der Probe erstreckt, wobei die Ebene der Probe bezüglich der Objektbrennebene versetzt ist.

## Claims

1. Method for analysing microorganisms ($10_i$), the microorganisms being placed in a sample, the sample comprising a viability marker ($10_v$) that is able to modify an optical property of the microorganisms differently depending on whether they are dead or alive, the method comprising the following steps:

   a) illuminating the sample using a light source (11), the light source emitting an incident light wave (12) that propagates towards the sample (10) along a propagation axis (Z);

   b) acquiring, using an image sensor (16), an image ($I_0$) of the sample (10), which image is formed in a detection plane ($P_0$), the sample being placed between the light source (11) and the image sensor (16), the image being representative of an exposure light wave (14) to which the image sensor (16) is exposed under the effect of the illumination, the image containing interference patterns due to interference between a portion (12') of the incident light wave (12) transmitted by the sample and the diffraction of the incident light wave (12) by the microorganisms;

   the method being **characterized in that** it also comprises the following steps:

   c) determining radial positions ($x_i$, $y_i$) of various microorganisms ($10_i$) in a plane parallel to the detection plane, each radial position being associated with one microorganism;

   d) to the image ($I_0$) acquired in step b), applying a propagation operator (h), so as to compute at least one characteristic quantity *(M, <p)* of the exposure light wave (14), at each radial position ($x_i$, $y_i$) determined in step c), and at a plurality of distances (z) from the detection plane ($P_0$);

   e) forming a profile ($M_i(z)$, $\varphi_i(z)$) representing a variation in the characteristic quantity computed in step d) along an axis parallel to the propagation axis (Z) and passing through each radial position ($x_i$, $y_i$) determined in step c), each profile being associated with one microorganism ($10_i$);

   f) depending on each profile formed in step e), classifying between microorganisms that are dead and microorganisms that are alive.

2. Method according to Claim 1, comprising, following step f), a step g) of analysing the capability of the microorganism to divide, step g) comprising the following substeps for at least one microorganism considered to be alive in step f):

> gi) obtaining a first observation image ($I(t_1)$) of the sample, at a first time ($t_1$), the observation image comprising regions of interest ($ROI_i$) respectively associated with microorganisms ($10_i$) and detecting a region of interest ($ROI_i$) associated with said microorganism ($10_{i,a}$);
> gii) acquiring an image ($I_0(t_2)$) of the sample at a second time ($t_2$), the second time being subsequent to the first time, and obtaining a second observation image ($I(t_2)$) of the sample on the basis of the image ($I_0(t_2)$) of the sample acquired at the second time;
> giii) detecting, in the second observation image, a region of interest ($ROI_i$) corresponding to the microorganism ($10_{i,a}$);
> giv) comparing the regions of interest detected in substeps gi) and giii);
> gv) determining the capability of the microorganism to divide depending on the comparison performed in substep giv).

3. Method according to Claim 2, wherein substep gv) comprises identifying, among the living microorganisms ($10_{i,a}$), microorganisms ($10_{i,a2}$) that are viable but non-culturable.

4. Method according to Claim 2 or Claim 3, wherein, in substeps gi) and gii), the first observation image ($I(t_1)$) and the second observation image ($I(t_2)$) are obtained by applying a propagation operator ($h$) to an image ($I_0(t_1)$) acquired at the first time and to the image ($I_0(t_2)$) acquired at the second time.

5. Method according to any one of Claims 2 to 4, wherein, in substep gi), the first observation image ($I(t_1)$) is obtained from the image ($I_0$) acquired in step b).

6. Method according to any one of Claims 2 to 5, wherein the time interval ($\Delta t$) between the first time and the second time is comprised between 5 hours and 70 hours.

7. Method according to any one of the preceding claims, wherein, in step d), the propagation operator is applied to the image ($I_0$) acquired in step b), for a plurality of propagation distances, so as to obtain a stack of complex images.

8. Method according to any one of Claims 1 to 6, wherein step d) comprises the following substeps:

> di) applying a propagation operator (h) to the image ($I_0$) acquired in step b), so as to compute a complex image ($A_{ref}$), called the reference image, representative of the sample in a reference plane ($P_{ref}$);
> dii) applying a propagation operator ($h$) to the reference image ($A_{ref}$), so as to obtain complex images, called secondary complex images ($A_{ref,z}$), at various distances ($z$) from the reference plane ($P_{ref}$) along the propagation axis ($Z$), the secondary complex images and the reference image forming a stack of complex images;
> diii) determining a radial position ($x_i$, $y_i$) of microorganisms ($10_i$) on the basis of the images of the stack of complex images obtained in substep dii).

9. Method according to any one of the preceding claims, wherein the viability marker ($10_v$) induces a colouration of the microorganisms when they are dead, in a colouration spectral band ($\Delta\lambda'$), and wherein, in step a), the sample is illuminated in an illumination spectral band ($\Delta\lambda$), the illumination spectral band not comprising all or some of the colouration spectral band.

10. Method according to any one of the preceding claims, wherein, in step d), the characteristic quantity is determined on the basis of the modulus or of the phase of the exposure light wave, at each distance from the detection plane.

11. Method according to any one of the preceding claims, wherein step f) comprises classifying each profile ($M_i(z)$, $\varphi_i(z)$) into a first class, corresponding to profiles characteristic of living microorganisms ($10_{i,a}$), and into a second class, corresponding to profiles characteristic of dead microorganisms ($10_{i,d}$).

12. Method according to Claim 11, wherein each profile ($M_i(z)$, $\varphi_i(z)$) is classified depending on its shape or on a maximum value or on a minimum value of the profile.

13. Method according to any one of the preceding claims, wherein no image-forming optic is placed between the sample and the image sensor.

14. Method according to any one of claims 1 to 12, wherein an image-forming optical system is placed between the sample and the image sensor, the optical system having an object focal plane, the sample lying in a plane of the sample, the plane of the sample being offset with respect to the object focal plane.

**Fig. 1**

**Fig. 2**

**Fig. 3A**

**Fig. 3B**

**Fig. 3C**

**Fig. 3D**

**Fig. 4A**

$10_{i,a}$      $10_{i,d}$

**Fig. 4B**

$10_{i,a1}$      $10_{i,a2}$   $10_{i,d}$

**Fig. 4C**

## RÉFÉRENCES CITÉES DANS LA DESCRIPTION

### Documents brevets cités dans la description

- WO 2016151249 A
- WO 2016151248 A
- WO 2016097092 A
- WO 9855861 A1
- US 20120218379 A
- FR 1554811
- FR 1652500

### Littérature non-brevet citée dans la description

- **FEIZI.** Rapid, portable and cost effective yeast cell viability and concentration analysis using lensfree on-chip microscopy and machine learning. *Lab Chip,* 2016, vol. 16, 4350-4358
- **KWOLEK-MIREK M.** comparison of methods used for assessing the viability and vitality of yeast cells. *FEMS Yeast Res,* 2014, vol. 14, 1068-1079
- **S. N. A. MOREL ; A. DELON ; P. BLANDIN ; T. BORDY ; O. CIONI ; L. HERVÉ ; C. FROMENTIN ; J. DINTEN ; C. ALLIER.** Wide-Field Lensfree Imaging of Tissue Slides. *Advanced Microscopy Techniques,* vol. IV
- Neurophotonics II. SPIE Proceedings. Optical Society of America, 2015, vol. 9536